**Europäisches Patentamt**

**European Patent Office**

**Office européen des brevets**

(11) Veröffentlichungsnummer: **0 205 133**

**A1**

# EUROPÄISCHE PATENTANMELDUNG

(21) Anmeldenummer: **86107790.7**

(22) Anmeldetag: **07.06.86**

(51) Int. Cl.⁴: **A 61 K 7/16**
**A 23 G 3/30, A 61 K 7/26**
**A 61 K 9/68**

(30) Priorität: **14.06.85 CH 2536/85**

(43) Veröffentlichungstag der Anmeldung:
**17.12.86 Patentblatt 86/51**

(84) Benannte Vertragsstaaten:
**DE FR GB**

(71) Anmelder: **Schicht, Marie Luise, Dr.**
**c/o Longeraie 3**
**CH-1005 Lausanne(CH)**

(72) Erfinder: **Schicht, Marie Luise, Dr.**
**c/o Longeraie 3**
**CH-1005 Lausanne(CH)**

(74) Vertreter: **Blum, Rudolf Emil Ernst et al,**
**c/o E. Blum & Co Patentanwälte Vorderberg 11**
**CH-8044 Zürich(CH)**

(54) **Plastische Kaumasse auf Basis einer Kaugummigrundmasse.**

㉡ Die plastische Kaumasse auf Basis einer Kaugummigrundmasse enthält ein basisches Mineralsalzkonzentrat und wenigstens einen pflanzlichen und/oder biologischen Wirkstoff.

Die erfindungsgemässe Kaumasse kann

1. als reinigendes Mund- und Zahnpflegemittel zum Schutz der Zähne, des Parodontiums und der Mundschleimhaut, und/oder

2. als biologisches und völlig unschädliches Raucherentwöhnungsmittel, und/oder

3. als biologisches und völlig unschädliches Mittel zur Steigerung der Vitalität und der Leistungsfähigkeit verwendet werden.

- 1 -

Plastische Kaumasse auf Basis einer Kaugummigrundmasse

------------------------------------------------

Die vorliegende Erfindung betrifft eine plastische Kaumasse auf Basis einer Kaugummigrundmasse.

Die Beobachtung von Naturvölkern hat gezeigt,
dass diese ihr gesundes Gebiss bis ins hohe Alter nicht
allein natürlicheren Ernährungsbedingungen, sondern auch
der Gewohnheit verdanken, regelmässig Baumharze oder harzähnliche Pflanzenbestandteile zu kauen. Diese durch intensiven Kauvorgang bedingte natürliche Mund- und Zahnreinigung ist eine notwendige Ergänzung der bei uns üblichen Zahnreinigungsmethoden, wobei ein überwiegender
Prozentsatz der Bevölkerung und in Hauptsache Kinder nur
hin und wieder die Zahnbürste verwenden.

Bei diesen Verfahren wird aus einem Percolat und
einem Macerat ein Gemisch gebildet, welches in eine Kaugummigrundmasse eingearbeitet wird. Es regt während des
Kauvorganges die Speichelsekretion an und bewirkt damit
die Durchspülung und Reinigung der Mundhöhle bzw. der
Zahnzwischenräume und der Zahnschmelzflächen.

Der Erfindung liegt die Erkenntnis zugrunde,
dass die Entstehung der Zahnkaries ein komplexer Vorgang
ist, an dem sowohl endogene als auch exogene Faktoren
massgeblich beteiligt sind.

Unter den exogenen Faktoren ist die zugeführte
Nahrung insbesondere hinsichtlich Gehalt und Qualität der Kohlehydrate von erheblicher Bedeutung. Leicht gärfähige isolierte Industrie-Zuckerarten und Feinmehle führen besonders beim Kariesanfälligen zu einer pathologischen Verschiebung des Kohlehydratstoffwechsels, zur anaerob-glykolytischen Säurebildung anstelle des oxydativen Abbaues mit Entstehung eines pathologisch sauren Speichels und zu einer pathologisch sauren Bakterienflora des Mundes, die zusammen mit der Verschiebung des Säure-Basengleichgewichtes im Organismus zur Entkalkung und Zerstörung der Zähne führt.

Der pathologische Abbau der Kohlehydrate ist keineswegs nur örtlich auf den Bereich der Mundhöhle beschränkt, sondern muss im Rahmen der gleichzeitig bestehenden Gesamtstoffwechselstörung, der gestörten Zellatmung und der pathologischen Verschiebung des Säure-Basenhaushaltes in Richtung Säureüberschuss gesehen werden. Eine latente Azidose geht immer mit einer vermehrten Kalkausscheidung und erhöhten Kariesanfälligkeit einher, während eine Normalisierung des Säure-Basenhaushaltes die Kariesbereitschaft eindeutig und augenblicklich herabsetzt.

Der erhebliche Säureüberschuss unserer modernen Zivilisationskost steht in engem Zusammenhang mit einem Mangel an Mineralsalzen, Vitaminen und Spurenelementen. Bei Anwendung der plastischen Kaumasse nach der Erfindung konnte nachgewiesen werden, dass bei Zufuhr der erforderlichen Mineralsalze, Vitamine und Spurenelemente in biologisch gegebener Relation eine Karies, selbst bei ausgesprochen kariesfördernder Ernährung weitgehend verhütet werden kann.

Ein weiterer exogener Faktor für die Entstehung der Karies ist die Tatsache, dass die gebräuchliche Koch- und Weichkost den für die natürliche Mund- und Zahnreinigung erforderlichen intensiven Kauprozess überflüssig macht. Gutes Kauen fördert die Sekretion von Spülspeichel, der für die vollständige Entfernung von Nahrungsresten, insbesondere aus den Interdentalräumen, von Bedeutung ist und im Gegensatz zur pathologischen Mundspeichelflora immer eine alkalische Reaktion aufweist.

Es ist ferner allgemein bekannt, dass der Wunsch oder Zwang zu rauchen im wesentlichen durch die Stresssituation des Alltags und die sympathikotone Reaktionslage des Rauchers mit dem Verlangen nach Entspannung verursacht wird. Stress und die Ueberfunktion des Sympathikus im vegetativen Nervensystem verkrampft und verengt die Blutgefässe und macht nervös. Rauchen entspannt und beruhigt durch Lähmung des Sympathikus vorübergehend und hat dann durch Provokation der überschiessenden Gegenregulation die gegenteilige Wirkung, die wiederum zum Rauchen zwingt.

Die meisten bekannten Raucherentwöhnungsmittel enthalten chemische Substanzen, die der Zigarette einen unangenehmen Geschmack verleihen. Sie sind zwar in der Lage, kurzfristig das Rauchen zu verhindern, vermögen aber nicht, die Ursache für die Sucht zu Rauchen zu beseitigen und innerhalb kürzester Zeit wird der Raucher diese Mittel nicht mehr verwenden und wieder nach seiner so geliebten Zigarette greifen, da er die entspannende Wirkung dessen nicht entbehren kann.

Der Erfindung liegt die Erkenntnis zugrunde, dass man nur dann einen Raucher von seiner Sucht befreien kann, wenn er etwas hat, welches ihm das gleiche Gefühl der Entspannung und Beruhigung bringt, wie der Genuss einer Zigarette. Nur in diesem Fall verliert er das Bedürfnis zu rauchen.

Die Beobachtung von Naturvölkern hat gezeigt, dass diese ihre erstaunliche Vitalität und Leistungsfähigkeit nicht allein ihren natürlichen Lebensbedingungen verdanken, sondern auch der Gewohnheit, regelmässig harzähnliche und andere Pflanzenbestandteile zu kauen, die aus dem Holz der Wurzelrinde und den Blättern bestimmter tropischer Pflanzen gewonnen werden.

Neuere wissenschaftliche Untersuchungen konnten die ausserordentliche Wirksamkeit dieser anregenden Pflanzenextrakte vollauf bestätigen und dieser moderne Fitmacher enthält verschiedene dieser wichtigen Pflanzenauszüge.

In bekannten Aufputschmitteln wird der Blutzuckerspiegel innerhalb kürzester Zeit erhöht, um dann plötzlich wieder auf ein niedriges Niveau abzusinken. Viele Verkehrsunglücke werden auf diese Weise verursacht.

Der Erfindung liegt die Erkenntnis zugrunde, dass spezifische, natürliche Wirkstoffe, welche nach und nach in der Mundhöhle absorbiert werden, einen ansteigenden und anhaltenden Effekt auf den gesamten Organismus ausüben, wobei es niemals zu einem plötzlichen und gefährlichen Absinken der Vitalität kommen kann.

Als zum Stand der Technik gehörend sei auch die DE-PS 1 049 544 erwähnt.

Dieser Erfindung liegt die Aufgabe zugrunde, die genannten Nachteile zu überwinden, und zwar mittels einer neuen plastischen Kaumasse. Diese Kaumasse soll zudem leicht und einfach hergestellt werden können.

Die erfindungsgemässe Kaumasse kann

1. als reinigendes Mund- und Zahnpflegemittel zum Schutz der Zähne, des Paradontiums und der Mundschleimhaut, und/oder

2. als biologisches und völlig unschädliches Raucherentwöhnungsmittel, und/oder

3. als biologisches und völlig unschädliches Mittel zur Steigerung der Vitalität und der Leistungsfähigkeit verwendet werden.

Wie oben aufgezeigt, ist auch die Idee, die erfingungsgemässe Kaumasse in mehr als einem Anwendungsbereich - nämlich in zwei und drei Anwendungsbereichen - gleichzeitig zum Einsatz zu bringen, völlig neu.

Die Erfindung ist durch die Merkmale in den unabhängigen Ansprüchen gekennzeichnet.

Eine erste bevorzugte Ausführungsform der Erfindung betrifft eine plastische Kaumasse als reinigendes Mund- und Zahnpflegemittel.

Sie ermöglicht durch die Art ihrer Anwendung und infolge ihrer spezifischen Zusammensetzung eine schnelle und sichere Zahnreinigung und schützt durch die örtliche Wirkung und durch die Resorption ihrer biologisch wirksamen Inhaltstoffe die Mundschleimhaut, den Zahn und das Parodontium vor schädigenden Einflüssen von innen und aussen.

Zwecks wirksamer Bekämpfung der Karies zeigt die Erfindung eine plastische Kaumasse als reinigendes Mund- und Zahnpflegemittel zum Schutz der Zähne, des Parodontiums und der Mundschleimhaut, bei dem zwecks Wiederherstellung des Säure-Basengleichgewichts im gesamten Organismus und zur Remineralisation des entkalkten Zahnes in eine an sich bekannte Kaugrundmasse ein basisches Mineralsalzkonzentrat eingearbeitet ist. Das Mineralsalzkonzentrat besteht z.B. aus einer Knochenmehlverreibung, aus Meersalz, Calciumcarbonat und Natriumcarbonat. Ausser diesen, für die beabsichtigte Wirkung unbedingt erforderlichen Inhaltsstoffen enthält die Kaumasse mit besonderem Vorteil folgende Bestandteile in Kombination:

a) ein Konzentrat aus einer Knochenmehlverreibung, Meersalz, Calciumcarbonat und Natriumbicarbonat

b) pflanzliche Wirkstoffe in Tinkturform: gegen Karies (Angustura, Symphytum, Nasturtium und Cochlearia)

gegen Parodontose (Ratanhia, Myrrha, Iris, Salvia, Chamomilla und Nelkentinktur)

zur Anregung der Salivation (Rad. pyrethri) zur Steigerung der körpereigenen Abwehrkraft (Echinacea) und zur Verbesserung von Bekömmlichkeit, Stoffwechselleistung und Geschmack (Zingiber, Calamus, Curcuma und Angustura)

c) ätherische Oele und Resinoide mit durchblutungsfördernder Wirkung auf Zahnfleisch und Mundhöhle (Salbeiöl, Nelkenöl)

zur Anregung der Speichelsekretion (Essence de piment, Nelkenöl)

gegen Parodontose (Salbeiöl, Nelkenöl)

zur Verbesserung von Bekömmlichkeit, Stoffwechselleistung und Geschmack (Anisöl, Fenchelöl, Cassisöl)

zur Aromatisierung, Erfrischung und Desodorierung (Pfefferminzöl, Krauseminzöl, Menthol, Eucalyptusöl und Citronenöl)

zur Kompensierung der Nikotinwirkung nach dem Rauchen in geschmacklicher und gesundheitlicher Hinsicht (Pfefferminzöl, Menthol und Eucalyptusöl)

d) Vitamin in Form von Calcium-D-Pantothenat

e) Weichmacher und Binder in Form von nicht gärfähigen und nicht kariogenen zuckerähnlichen Verbindungen zur Geschmacksverbesserung und Kariesverhütung (Sorbit und Glycerin).

Zwecks Optimierung der angestrebten Wirkung sind die Inhaltsstoffe unter Beachtung etwa folgender Gewichtsanteile in bzw. mit der Kaugummigrundmasse eingearbeitet bzw. vermischt:

a) Kaubare Naturharze als Kaugummigrund-   25,0%
   masse

b) Knochenmehlverreibung   5,0%

c) Meersalz   1,5%

d) Calciumcarbonat   5,0%

e) Natriumbicarbonat   0,2%

f) Kräutertinktur   7,0%

aus Tinct. ratanhiae   55,0 g

     "     echinacea   8,8 g

     "     benzoe   8,8 g

     "     myrrhae   8,8 g

     "     iridis   8,9 g

     "     symphyti   12,0 g

     "     nasturtii   8,8 g

     "     cochleariae   8,8 g

     "     salviae   8,8 g

aus Tinct. chemomillae        8,8 g

"         zingiberis        11,0 g

"         calami            11,0 g

"         curcumae          11,0 g

"         angusturae         8,8 g

"         pytethri comm.     8,8 g

"         caryophylli       11,0 g

                             200,0 g

g) Calcium-D. Pantothenat                    0,1%

h) ätherische Oele                           1,5%

nämlich Pfefferminzöl Ia      600 g

Krauseminzöl                   25 g

Menthol recrist DAB 42/44°    120 g

Eucalyptusöl span.glob.
          rekt. 70/75 %        50 g

Anisöl amerik.                 50 g

Fenchelöl span.süss DAB        50 g

Salbeiöl span. EB              20 g

Nelkenöl gereinigt 80/85 %     20 g

Cassisöl rekt. 80/85 %         50 g

Citronenöl itl. DAB             5 g

Essence de piment              10 g

                             1000 g

i) nicht kariogener Zuckerersatz, wie z.B. Sorbit, Mannit oder dergleichen           53,9%

k) Glycerin                                0,5%

l) natürliche Farbstoffe                   0,3%.

Ein bevorzugtes Verfahren zur Herstellung einer plastischen Kaumasse gemäss der ersten bevorzugten Ausführungsform der Erfindung kann so ausgeführt werden, dass die Kaugummigrundmasse in an sich bekannter Weise in einem vorher erhitzten Mixer, z.B. vom Typ Baker Perkins, weich gemacht und sodann der nicht kariogene Zuckerersatz, wie Mannit, Sorbit, oder dergleichen, portionsweise hinzugefügt und jeweils in die Grundmasse eingearbeitet wird, worauf jeweils die übrigen Inhaltsstoffe zugemischt werden.

Es kann auch so verfahren werden, dass zwecks leichterer Verarbeitung zwischen den einzelnen Portionen des nicht kariogenen Zuckerersatzes eine kleine Menge Kräutertinktur eingearbeitet wird.

Es ist beispielsweise auch möglich, dass die Inhaltsstoffe in der Reihenfolge: nicht kariogener Zuckerersatz, Glycerin, Calciumcarbonat, Knochenmehlverreibung, kleine Menge Kräutertinktur, Meersalz, Natriumbicarbonat, natürliche Farbstoffe, Calcium-D-Pantothenat, Aromaöle und der Rest Kräutertinktur in die Kaugummigrundmasse eingearbeitet werden, wobei zwischen den einzelnen Portionen eines Inhaltsstoffes bzw. zwischen den jeweiligen Inhaltsstoffen eine innige Vermischung der Menge erfolgt.

Bei der Herstellung einer solchen plastischen Kaumasse ist es von besonderer Bedeutung, dass die Kaugummigrundmasse in an sich bekannter Weise in einem vorher erhitzten Mixer vom Typ Baker Perkins weich gemacht und sodann der nicht kariogene Zuckerersatz, wie Mannit, Sorbit oder dergleichen, portionenweise hinzugefügt und jeweils in die Grundmasse eingearbeitet wird, worauf jeweils die übrigen Inhaltsstoffe zugemischt werden. Zwecks leichterer Verarbeitung wird zwischen den einzelnen Portionen des nicht kariogenen Zuckerersatzes eine kleine Menge Kräutertinktur eingearbeitet.

Zweckmässig werden die Inhaltsstoffe in folgender Reihenfolge in die Kaugummigrundmasse eingearbeitet: nicht kariogener Zuckerersatz, Glycerin, Calciumcarbonat, Knochenmehlverreibung, kleine Menge Kräutertinktur, Meersalz, Natriumbicarbonat, natürliche Farbstoffe, Calcium-D-Pantothenat, Aromaöle und Rest Kräutertinktur. Dabei erfolgt zwischen der Zugabe der einzelnen Portionen eines Inhaltsstoffes bzw. zwischen den jeweiligen Inhaltsstoffen eine innige Vermischung der Masse. Im einzelnen wird die Kaugummigrundmasse in einem Mixer, z.B. vom Typ Baker Perkins, weich gemacht und der nicht kariogene Zuckerersatz beispielsweise in drei gleichen Portionen hinzugefügt. Dies erfolgt etwa im Abstand von 3-4 Minuten. Danach wird das Glycerin hinzugetan und vermischt. Nach 1-2 Minuten erfolgt die Zugabe von Calciumcarbonat und nach weiteren 2 Minuten die Zugabe der Knochenmehlverreibung und einer kleinen Menge Kräutertinktur. Durch die Kräutertinktur wird der Grundmasse eine gewisse Feuchtigkeit zugegeben, wodurch die Masse leichter knetbar wird. Anschliessend wird das Meersalz und das Natriumbicarbonat beigemischt. Nach einigen Minuten folgen die natürlichen Farbstoffe und das Calcium-D-Pantothenat. Nach einer weiteren Minute, die zum Mixen der Masse gebraucht wird, werden die Aromaöle hinzugefügt und sodann soviel Kräutertinktur, wie die Masse fasst. Anschliessend wird die Masse nach 3-4 Minuten innigst vermischt und gemixt, bevor sie aus dem Mixer entfernt wird. Die Temperatur der Masse liegt zu diesem Zeitpunkt etwa bei 55°C. Die plastische Kaumasse wird anschliessend portionsgerecht unterteilt und nach der Abkühlung entsprechend verpackt.

Das regelmässige Kauen der plastischen Kaumasse nach der Erfindung mit für den Kauapparat und die Mundhöhle spezifischen biologischen Wirkstoffen regt die bei den meisten Menschen mangelhafte Speichelsekretion lebhaft an und bewirkt eine gute Durchspülung der Mundhöhle, der Interdentalräume und eine ausgezeichnete mechanische Reinigung durch Saugwirkung der plastischen Masse auch in engen Interdentalräumen und an den Stellen, die von der üblichen Zahnbürste nicht erreicht werden können. Der intensive Kauvorgang regt die Durchblutung von Zahnfleisch und Zahnwurzel an, kräftigt die Kaumuskulatur und den Zahnhalteapparat und ist ein wirksames Mittel und Prophylaktikum gegen die Parodontose. Die Kaumasse als Zahnschutz- und Zahnpflegemittel enthält neben einer geeigneten palstischen Kaugummigrundmasse alle wesentlichen biologischen Wirkstoffe zur Gesunderhaltung des Zahnes, zur Kariesverhütung, gegen Erkrankung der Mundschleimhaut und gegen die Parodontose. Neben der örtlichen Einwirkung wird durch Re-

0205133

sorption dieser Wirkstoffe über die Mundschleimhaut eine Aktivierung körpereigener spezifischer Abwehrkräfte ausgelöst, die einen wirksamen Schutz der Mundhöhle - der Eingangspforte der meisten Infektionen -, des Zahnes des Parodontiums gegen schädigende und zerstörende Einwirkungen von innen und von aussen darstellt.

Bei Anwendung der plastischen Kaumasse nach der Erfindung konnte nachgewiesen werden, dass bei Zufuhr der erforderlichen Mineralsalze, Vitamine und Spurenelemente in biologisch gegebener Relation eine Karies, selbst bei ausgesprochen kariesfördernder Ernährung, weitgehend verhütet werden kann.

Ferner sei noch auf die folgenden Punkte hingewiesen:

1. In Anbetracht, dass ein Zahnmittel, welches zuviel Säure aufweist, den Zahnschmelz angreift und anderseits, wenn das Zahnmittel zu stark basisch ist, zuviel Kalk erzeugt wird und somit die Zähne bröcklig werden, ist eine Zielsetzung der neuen Rezeptur, dass eine enge Toleranz von Säure und Basis erzielt wird. Ein wichtiges Merkmal hierbei ist, den Säureüberschuss zu neutralisieren und innerhalb engen Toleranzen zu halten. Hierbei besteht eine weitere Zielsetzung in der Erzielung einer Langzeitwirkung, welche ursächlich für die wirkungsvolle Kariesbekämpfung ist. Die Inhaltsstoffe des Rezeptes sind gezielt gewählt, so dass das ideale Säuren-Basen-Gleichgewicht schneller erreicht wird und anhält.

2. Ein weiteres Merkmal der Rezepturmasse besteht in einer guten Saugkraft, durch welche es erst möglich wird, die Zähne gut zu reinigen durch Heraussaugen der Speiseresten, welche sich in den Zahnzwischenräumen befinden.

3. Durch die Rezeptur werden auch die Fabrikationsmöglichkeiten einfacher, bzw. es sind in der Herstellung weniger Operationen notwendig, um eine homogene Masse zu erreichen, im Gegensatz zu früher, da mehrere Operationen notwendig waren und die Homogenität infrage stand.

Eine zweite bevorzugte Ausführungsform der Erfindung betrifft eine plastische Kaumasse als biologisches und völlig unschädliches Raucherentwöhnungsmittel.

Die speziell ausgewählten biologisch wirksamen Substanzen ermöglichen durch die Art ihrer Anwendung und infolge ihrer spezifischen Zusammensetzung eine Beruhigung und Entkrampfung nach Stressituationen, so dass der Raucher kein Bedürfnis mehr empfindet zu rauchen. Durch Aktivierung des Stoffwechsels wird gleichzeitig der Nikotinspiegel im Blut gesenkt, so dass der Raucher nach der Entwöhnung nicht an Gewicht zunimmt.

Um den nervösen Beschäftigungsdrang des Rauchers - er muss etwas im Munde haben - entgegen zu kommen, wird das Entwöhnungsmittel bewusst in Form eines Kaupräparates angeboten. Erfahrungsgemäss vermag der Kauvorgang das Rauchen bis zu einem gewissen Grade zu ersetzen.

Um einen optimalen Zustand der Entspannung und Entkrampfung zu erreichen, findet man in dem Mittel eine Kombination von:

a) ein Konzentrat aus einer Knochenmehlverreibung, Meersalz, Calciumcarbonat und Natriumbicarbonat.

b) pflanzliche Wirkstoffe in homöopathischen Potenzen:

- gegen Krämpfe, bei Schwäche und Müdigkeit (Lobelia inflata = indischer Tabak)

- bei Erregungszuständen, Unruhe, Nervosität (Stramonium)

- gegen Stress, gegen Magenkrämpfe, gegen Schmerzen, Melancholie und Angst (Belladonna)

- bei Uebererregbarkeit und Kopfdruck (Anhalonium·Lewinii Henn.)

c) ätherische Oele und Resinoide mit durchblutungsfördernder Wirkung (Salbeiöl, Nelkenöl)

zur Anregung der Speichelsekretion und des Stoffwechsels (Essence de piment, Nelkenöl)

zur Verbesserung der Bekömmlichkeit, Stoffwechselleistung und Geschmack (Anisöl, Fenchelöl, Cassisöl)

zur Aromatisierung, Erfrischung und Desodorierung (Pfefferminzöl, Krauseminzöl, Menthol, Eucalyptusöl und Citronenöl).

d) Vitamin in Form von Calcium-D-Pantothenat.

e) Weichmacher und Binder in Form von nicht gärfähigen zuckerähnlichen Verbindungen zur Geschmacksverbesserung (Sorbit und Glycerin).

Zwecks Optimierung der angestrebten Wirkung sind die Inhaltsstoffe unter Beachtung etwa folgender Gewichtsanteile in bzw. mit der Kaugummigrundmasse eingearbeitet bzw. vermischt:

| | | |
|---|---|---|
| a) Kaubare Naturharze als Kaugummigrundmasse | | 25,0 % |
| b) Knochenmehlverreibung | | 5,0 % |
| c) Meersalz | | 1,5 % |
| d) Calciumcarbonat | | 5,0 % |
| e) Natriumbicarbonat | | 0,2 % |
| f) Pflanzliche Wirkstoffe: | | 7,0 % |
| Lobelia inflata | $D_{12}$ | |
| Stramonium | $D_{12}$ | |
| Belladonna | $D_{12}$ | |
| Anhalonium | $D_{12}$ | |

aa 75,0 g

| | | |
|---|---|---|
| g) Calcium-D-Pantothenat | | 0,1 % |
| h) ätherische Öle | | 1,5 % |
| nämlich Pfefferminzöl Ia | 600 g | |
| Krauseminzöl | 25 g | |
| Menthol reorist DAB 42/44° | 120 g | |
| Eucalyptusöl span. glob. | | |
| rekt. 70/75 % | 50 g | |
| Anisöl amerik. | 50 g | |
| Fenchelöl span. süss DAB | 50 g | |
| Salbeiöl span. EB | 20 g | |
| Nelkenöl gereinigt 80/85 % | 20 g | |

| Cassisöl rekt. 80/85 % | 50 g |
| Citronenöl itl. DAB | 5 g |
| Essence de piment | 10 g |
| | 1000 g |

i) nicht gärfähiger Zuckerersatz,
   wie z.B. Sorbit, Mannit od.dgl.      53,9 %

k) Glycerin      0,5 %

l) natürliche Farbstoffe      0,3 %.

Ein bevorzugtes Verfahren zur Herstellung einer plastischen Kaumasse gemäss der zweiten bevorzugten Ausführungsform der Erfindung kann so ausgeführt werden, dass die Kaugummigrundmasse in an sich bekannter Weist in einem vorher erhitzten Mixer, z.B. vom Typ Baker Perkins, weich gemacht und sodann der nicht gärfähige Zuckerersatz, wie Mannit, Sorbit, oder dergleichen, portionenweise hinzugefügt und jeweils in die Grundmasse eingarbeitet wird, worauf jeweils die übrigen Inhaltstoffe zugemischt werden.

Es kann auch so verfahren werden, dass zwecks leichterer Verarbeitung zwischen den einzelnen Portionen des nicht gärfähigen Zuckerersatzes eine kleine Menge der pflanzlichen Wirkstoffe eingearbeitet wird.

Es ist beispielsweise auch möglich, dass die Inhaltstoffe in der Reihenfolge nicht gärfähiger Zuckerersatz, Glycerin, Calciumcarbonat, Knochenmehlverreibung, kleine Menge der pflanzlichen Wirkstoffe, Meersalz, Natriumcarbonat, natürliche Farbstoffe, Calcium-D-Pantothenat, Aromaöle und der Rest der pflanzlichen Wirkstoffe in die Kaugummigrundmasse eingearbeitet werden, wobei zwischen den jeweiligen Inhaltstoffen eine innige Vermischung der Menge erfolgt.

Bei der Herstellung einer solchen plastischen Kaumasse ist es von besonderer Bedeutung, dass die Kaugummigrundmasse in an sich bekannter Weise in einem vorher erhitzten Mixer, z.B. vom Typ Baker Perkins, weich gemacht und sodann dem nicht gärfähigen Zuckerersatz, wie Mannit, Sorbit oder dergleichen, portionenweise hinzugefügt und jeweils in die Grundmasse eingearbeitet wird, worauf jeweils die übrigen Inhaltstoffe zugemischt werden. Zwecks leichterer Verarbeitung wird zwischen den einzelnen Portionen des nicht gärfähigen Zuckerersatzes eine kleine Menge der pflanzlichen Wirkstoffe eingearbeitet.

Zweckmässig werden die Inhaltstoffe in folgender Reihenfolge in die Kaugummigrundmasse eingearbeitet:

Nicht gärfähiger Zuckerersatz, Glycerin, Calciumcarbonat, Knochenmehlverreibung, kleine Menge der pflanzlichen Wirkstoffe, Meersalz, Natriumbicarbonat, natürliche Farbstoffe, Calcium-D-Pantothenat, Aromaöle und Rest der pflanzlichen Wirkstoffe. Dabei erfolgt zwischen der Zugabe der einzelnen Portionen eines Inhaltstoffes bzw. zwischen den jeweiligen Inhaltstoffen eine innige Vermischung der Masse.

Im einzelnen wird die Kaugummigrundmasse in einem Mixer, z.B. vom Typ Baker Perkins, weich gemacht und der nicht gärfähige Zuckerersatz beispielsweise in drei gleichen Portionen hinzugefügt.

Dies erfolgt etwa im Abstand von 3-4 Minuten. Danach wird das Glycerin hinzugegeben und vermischt. Nach 1-2 Minuten erfolgt die Zugabe von Calciumcarbonat und nach weiteren 2 Minuten die Zugabe der Knochenmehlverreibung und einer kleinen Menge der pflanzlichen Wirkstoffe. Durch diese Wirkstoffe wird der Grundmasse eine gewisse Feuchtigkeit zugegeben, wodurch die Masse leichter knetbar wird.

Anschliessend wird das Meersalz und das Natriumbicarbonat beigemischt. Nach einigen Minuten folgen die natürlichen Farbstoffe und das Calcium-D-Pantothenat. Nach einer weiteren Minute, die zum Mixen der Masse gebraucht wird, werden die Aromaöle hinzugefügt und dann soviel der pflanzlichen Wirkstoffe, wie die Masse fasst. Anschliessend wird die Masse noch 3-4 Minuten innigst vermischt und gemixt, bevor sie aus dem Mixer entfernt wird. Die Temperatur der Masse liegt zu diesem Zeitpunkt etwa bei 55°C.

Die plastische Kaumasse wird anschliessend portionsgerecht unterteilt und nach der Abkühlung entsprechend verpackt.

Die Durchblutung der Mundhöhle und des ganzen Kopfes bessert sich, einerseits durch den intensiven Kauvorgang und andererseits durch die spezifischen pflanzlichen Wirkstoffe, welche speziell zu diesem Zweck gewählt worden sind.

Die Lobelia inflata wurde schon im Jahre 1813 von Culter zur Entspannung bei der Asthma-Therapie verwendet. Auch Reece verwendete es bei Asthmatikern zur Entkrampfung.

In der Homöopathie verwendet man Lobelia u.a. bei Verkrampfungen, Hysterie, Nervenüberreizung, Enzephalitis usw.

Stramonium wurde durch Störck zum ersten Male in 1762 in Deutschland in Gebrauch genommen wegen seiner guten Wirkung bei Krämpfen, Epilepsie und Wahnsinn. Hufeland verwendete es bei Asthma, Verkrampfung und sogar Geistesgestörtheit. Bentley lobte es als schmerz- und krampflösendes Mittel. Hahnemann verwendete es bei Veitstanz und bei Melancholie.

Heute wird Stramonium in der Homöopathie hauptsächlich bei starker Hirnreizung, bei Krampfbereitschaft und zur Behandlung des vegetativen Nervensystems öfters verwendet.

Belladonna ist wohl einer der bekanntesten Heilpflanzen. In 1814 wurde sie von Hecker als schmerz- und krampfstillendes Mittel angeführt. Man verwendete es bei Nerven- und Gemütskrankheiten, bei Epilepsie und Chorea zur Erleichterung und Minderung des Leidens. Auch Hufeland verwendete es oft als Antispasmodikum und Gehirnmittel. Hahnemann heilte mit Belladonna viele Arten von Manie und Melancholie. Heute verwendet man Belladonna in der Homöopathie bei vielen Krankheiten, wie Manie, Epilepsie, akuten Neuralgien, Kopfschmerzen usw. Es ist ein gutes
Mittel bei Postenzephalitis der Parkinsonschen Krankheit.

Anhalonium lewinii. In Mexico ist diese Pflanze
schon sehr lange bekannt. Die Mexikaner lobten sie wegen
ihrer Kraft und Mut gebenden Wirkung. Anhalonium wird in
der Homöopathie empfohlen bei Gehirnerschöpfung und Koordinationsstörungen, sowie bei peripheren Durchblutungsstörungen.

Diese speziell gewählten pflanzlichen Wirkstoffe
entspannen die verkrampften Nerven und Blutgefässe, so
dass Kopfschmerzen, Kopfdruck und andere durch Verkrampfung entstandene Beschwerden nach und nach verschwinden.

Um dem nervösen Beschäftigungsdrang des Rauchers
entgegen zu kommen wird das Präparat bewusst als Kaupräparat angeboten.

Diese Erfindung ist ein vollwertiger Ersatz für
Tabakgenuss ohne die gefährlichen Folgen.

Eine dritte bevorzugte Ausführungsform der Erfindung betrifft eine plastische Kaumasse als biologisches
und völlig unschädliches Mittel zur Steigerung der Vitalität und der Leistungsfähigkeit.

Durch die Art der Anwendung dieser Kaumasse und
infolge ihrer spezifischen Zusammenstellung ermöglicht sie
eine Vitalisierung und eine Zunahme der Leistungsfähigkeit
und des Stoffwechsels. Als erfreuliche Nebenwirkung kann
ein beschleunigter Abbau des Blutalkoholgehaltes und von
störenden Fettpolstern konstatiert werden.

Die pflanzlichen Wirkstoffe in diesem Kaupräparat bauen verlorengegangene körperliche und geistige Kraftreserven nach und nach wieder auf. Es steigert die Vitalität und erfrischt anhaltend. Man nimmt es immer dann, wenn
man fit und leistungsfähig sein will oder muss, beim Autofahren, bei der Arbeit, bei Tagungen, am Morgen, am Abend
oder in der Nacht. Je nach Bedarf.

Um eine optimale tonisierende und entspannende
Wirkung zu erreichen, findet man in dem Mittel eine Kombination von:

a) ein Konzentrat aus einer Knochenmehlverreibung, Meersalz, Calciumcarbonat und Natriumbicarbonat;

b) pflanzliche und biologische Wirkstoffe in
homöopathischen Potenzen:

- gegen Schwäche und zur Beruhigung (Ferrum phosphoricum)

- bei Depressionen und Müdigkeit (Kalium phosphoricum)

- bei zu schneller Ermüdung und Abgespanntheit
(Semen colae)

- als Tonikum bei Schwächegefühl (Muira puama)

c) ätherische Oele und Resinoide mit durchblutungsfördernder Wirkung (Salbeiöl, Nelkenöl),

zur Anregung der Speichelsekretion und des
Stoffwechsels (Essence de piment, Nelkenöl),

zur Verbesserung der Bekömmlichkeit, Stoffwechselleistung und Geschmack (Anisöl, Fenchelöl, Cassisöl),

zur Aromatisierung, Erfrischung und Desodorierung (Pfefferminzöl, Krauseminzöl, Menthol, Eucalyptusöl
und Citronenöl).

d) Vitamin in Form von Calcium-D-Pantothenat.

e) Weichmacher und Binder in Form von nicht gärfähigen zuckerähnlichen Verbindungen zur Geschmacksverbesserung (Sorbit und Glycerin).

Zwecks Optimierung der angestrebten Wirkung sind
die Inhaltsstoffe unter Beachtung etwa folgender Gewichtsanteile in bzw. mit der Kaugummigrundmasse eingearbeitet
bzw. vermischt:

a) Kaubare Naturharze als Kaugummigrundmasse     25,0 %

b) Knochenmehlverreibung     5,0 %

c) Meersalz     1,5 %

d) Calciumcarbonat     5,0 %

e) Natriumbicarbonat     0,2 %

f) Pflanzliche- und biologische Wirkstoffe     7,0 %

Ferrum phos.      $D_1$

Kalium phos.      $D_1$

Semen colae       $D_{12}$

Muira puama       $D_{12}$

_____

aa  75,0 g

g) Calcium-D-Pantothenat                              0,1 %

h) ätherische Öle                                     1,5 %

  nämlich Pfefferminzöl Ia          600 g

  Krauseminzöl                       25 g

  Menthol reorist DAB 42/44°        120 g

  Eucalyptusöl span.glob.

             rekt. 70/75 %           50 g

  Anisöl amerik.                      50 g

  Fenchelöl span. süss DAB            50 g

  Salbeiöl span. EB                   20 g

  Nelkenöl gereinigt 80/85 %          20 g

  Cassisöl rekt. 80/85 %              50 g

  Citronenöl itl. DAB                  5 g

  Essence de piment                   10 g

                                  _____

                                   1000 g

i) nicht gärfähiger Zuckerersatz,                    53,9 %

   wie z.B. Sorbit, Mannit od. dgl.

k) Glycerin                                           0,5 %

l) natürliche Farbstoffe                              0,3 %.

Ein bevorzugtes Verfahren zur Herstellung einer plastischen Kaumasse gemäss der dritten bevorzugen Ausführungsform der Erfindung kann so ausgeführt werden, dass die Kaugummigrundmasse in an sich bekannter Weise in einem vorher erhitzten Mixer, z.B. vom Typ Baker Perkins, weich gemacht und sodann der nicht gärfähige Zuckerersatz, wie Mannit, Sorbit oder dergleichen, portionsweise hinzugefügt und jeweils in die Grundmasse eingearbeitet wird, worauf jeweils die übrigen Inhaltsstoffe zugemischt werden.

Es kann auch so verfahren werden, dass zwecks

- 19 -

leichterer Verarbeitung zwischen den einzelnen Portionen des nicht gärfähigen Zuckerersatzes eine kleine Menge der pflanzlichen und biologischen Wirkstoffe eingearbeitet wird.

Es ist beispielsweise auch möglich, dass die Inhaltsstoffe in der Reihenfolge nicht gärfähiger Zuckerersatz, Glycerin, Calciumcarbonat, Knochenmehlverreibung, kleine Menge der pflanzlichen Wirkstoffe, Meersalz, Natriumcarbonat, natürliche Farbstoffe, Calcium-D-Pantothenat, Aromaöle und der Rest der pflanzlichen und biologischen Wirkstoffe in die Kaugummigrundmasse eingearbeitet werden, wobei zwischen den jeweiligen Inhaltsstoffen eine innige Vermischung der Menge erfolgt.

Bei der Herstellung einer solchen plastischen Kaumasse ist es von besonderer Bedeutung, dass die Kaugummigrundmasse in an sich bekannter Weise in einem vorher erhitzten Mixer, z.B. vom Typ Baker Perkins, weich gemacht und sodann dem nicht gärfähigen Zuckerersatz, wie Mannit, Sorbit oder dergleichen, portionsweise hinzugefügt und jeweils in die Grundmasse eingearbeitet wird, worauf jeweils die übrigen Inhaltstoffe zugemischt werden. Zwecks leichterer Verarbeitung wird zwischen den einzelnen Portionen des nicht gärfähigen Zuckerersatzes eine kleine Menge der pflanzlichen und biologischen Wirkstoffe eingearbeitet.

Zweckmässig werden die Inhaltstoffe in folgender Reihenfolge in die Kaugummigrundmasse eingearbeitet:

Nicht gärfähiger Zuckerersatz, Glycerin, Calciumcarbonat, Knochenmehlverreibung, kleine Menge der pflanzlichen und biologischen Wirkstoffe, Meersalz, Natriumbicarbonat, natürliche Farbstoffe, Calcium-D-Pantothenat, Aromaöle und Rest der pflanzlichen und biologischen Wirkstoffe. Dabei erfolgt zwischen der Zugabe der einzelnen Portionen eines Inhaltstoffes bzw. zwischen den jeweiligen Inhaltstoffen eine innige Vermischunt der Masse.

Im einzelnen wird die Kaugummigrundmasse in einem Mixer, z.B. vom Typ Baker Perkins, weich gemacht und der nicht gärfähige Zuckerersatz beispielsweise in drei gleichen Portionen hinzugefügt.

Dies erfolgt etwa im Abstand von 3-4 Minuten. Danach wird das Glycerin hinzugegeben und vermischt. Nach 1-2 Minuten erfolgt die Zugabe von Calciumcarbonat und nach weiteren 2 Minuten die Zugabe der Knochenmehlverreibung und einer kleinen Menge der pflanzlichen und biologischen Wirkstoffe. Durch diese Wirkstoffe wird der Grundmasse eine gewisse Feuchtigkeit zugegeben, wodurch die Masse leichter knetbar wird. Anschliessend wird das Meersalz und das Natriumbicarbonat beigemischt. Nach einigen Minuten folgen die natürlichen Farbstoffe und das Calcium-D-Pantothenat. Nach einer weiteren Minute, die zum Mixen der Masse gebraucht wird, werden die Aromaöle hinzugefügt und dann soviel der pflanzlichen und biologischen Wirkstoffe, wie die Masse fasst. Anschliessend wird die Masse noch 3-4 Minuten innigst vermischt und gemixt, bevor sie aus dem Mixer entfernt wird. Die Temperatur der Masse liegt zu diesem Zeitpunkt etwa bei 55°C.

Die plastische Kaumasse wird anschliessend portionsgerecht unterteilt und nach der Abkühlung entsprechend verpackt.

Die Durchblutung der Mundhöhle und des ganzen Kopfes bessert sich, einerseits durch den intensiven Kauvorgang und andererseits durch die spezifischen pflanzlichen und biologischen Wirkstoffe, welche speziell zu diesem Zweck gewählt worden sind.

Das Ferrum phosphoricum - das phosphorsaure Eisen wird schon seit sehr lange in der Homöopathie verwendet. Bei Erschöpfungszuständen nach Infektionskrankheiten hat man sehr gute Erfolge damit gehabt. Es hilft dabei mit, die verlorengegangene Energie zurückzugewinnen. Oft wird in der Homöopathie die 6-12. Verreibung gegeben.

Auch Kalium phosphoricum hilft gegen Schwäche, Müdigkeit und Depressionen. Man gibt es gegen Tagesschläf-

- 21 -

rigkeit, Reizbarkeit und bei allgemeiner Unruhe. Es ist ein gutes Nervenmittel und wird in der Homöopathie meistens in der $D_6$ verschrieben.

Semen colae - die Kolanuss - wurde 1880 in Europa als Arzneimittel und Genussmittel eingeführt. Kolanüsse enthalten bis zu 3,5 % Koffein und haben eine ähnliche Wirkung wie Coffea, doch weniger stark. In der Homöopathie verwendet man es z.B. im Wechsel mit China gegen allgemeine Schwäche. Colae belebt und lässt Müdigkeit verschwinden.

Muira puama - das Potenzholz - wird in der Homöopathie als Tonikum gegen Schwäche verwendet. Es ist in seiner Heimat Süd-Amerika schon seit dem Altertum ein sehr beliebtes Heilmittel. In Europa wurde es vor etwa 80 Jahren eingeführt. Es ist ein ausgezeichnetes Roburans.

Diese speziell ausgewählte biologische Substanzen in hoher homöopathischer Dilution kompensieren sich gegenseitig. Sie entspannen und beleben zugleich. Müdigkeit verschwindet und man fühlt sich wieder leistungsfähig.

Die in diesem Mittel enthaltenen Vitalstoffe, ätherische Oele und tonisierende pflanzliche und biologische Wirkstoffe wirken in Verbindung mit den ebenfalls inkorporierten essentiellen Phosphatiden natürlicher Herkunft und den pflanzlichen zuckerähnlichen Verbindungen zuverlässig vitalisierend und leistungssteigernd. Sie aktivieren den Stoffwechsel, so dass es zu einer erhöhten Verbrennung von Stoffwechselrückständen, Kohlehydraten, Fetten und Alkohol kommt. So kann als erfreuliche Nebenwirkung ein beschleunigter Abbau des Blutalkoholgehaltes und von störenden Fettpolstern konstatiert werden. Unangenehmer Mundgeruch und Alkoholfahne verschwinden sofort. Durch den andauernden Kauvorgang werden die Wirkstoffe im wesentlichen bereits in der Mundhöhle resorbiert, so dass eine langsam sich steigernde anhaltende Wirkung erzielt wird.

Das Kaupräparat ist nicht mit Glucose gesüsst und kann deshalb auch von Diabetikern unbedenklich genommen werden.

Es sei noch..als darauf hingewiesen, dass die drei obe ..... bevorzugten Ausführungsformen sowohl in beliebiger Art und Weise miteinander kombiniert als auch je einzeln eingesetzt werden können. Es gibt somit sieben verschiedene Einsatzmöglichkeiten:

1. Zahnpflegemittel
2. Raucherentwöhnungsmittel
3. Vitalitätsmittel
4. Zahnpflege- und Raucherentwöhnungsmittel
5. Zahnpflege- und Vitalitätsmittel
6. Raucherentwöhnungs- und Vitalitätsmittel
7. Zahnpflege- und Raucherentwöhnungs- und Vitalitätsmittel.

Patentansprüche
----------------

1. Plastische Kaumasse auf Basis einer Kaugummigrundmasse, dadurch gekennzeichnet, dass sie ein basisches Mineralsalzkonzentrat und wenigstens einen pflanzlichen und/oder biologischen Wirkstoff enthält.

2. Kaumasse nach Anspruch 1, dadurch gekennzeichnet, dass das Mineralsalzkonzentrat aus einer Kno-chenmehlverreibung, Meersalz, Calciumcarbonat und Natrium-bicarbonat besteht.

3. Kaumasse nach einem der Ansprüche 1 bis 2, dadurch gekennzeichnet, dass sie noch wenigstens eine weitere Substanz, ausgewählt aus der Gruppe, bestehend aus Spurenelementen, ätherischen Oelen, Resinoiden, Weich-machern, Bindern, Geschmackstoffen, Geruchstoffen, natür-lichen und künstlichen Süssmitteln, Farbstoffen und Hilfsstoffen, enthält.

4. Kaumasse nach einem der Ansprüche 1 bis 3, dadurch gekennzeichnet, dass sie das basische Mineralsalzkonzentrat in einer Menge von 2 bis 50 Gew.-%, vorzugsweise von 5 bis 45 Gew.-%, und insbesondere von 8 bis 30 Gew.-%, und den Wirkstoff in einer Menge von 1 bis 30 Gew.-%, vorzugsweise von 2 bis 25 Gew.-%, und insbesondere von 5 bis 20 Gew.-%, enthält.

5. Kaumasse nach einem der Ansprüche 1 bis 4, dadurch gekennzeichnet, dass sie folgende Bestandteile enthält:

a) ein Konzentrat aus einer Knochenmehlverreibung, Meersalz, Calciumcarbonat und Natriumbicarbonat,

b) pflanzliche Wirkstoffe in Tinkturform:

- gegen Karies, z.B. Angustura, Symphytum, Nasturtium und Cochlearia,

- gegen Parodontose, z.B. Ratanhia, Myrrhia, Iris, Salvia, Chamomilla und Nelkentinktur,

- zur Anregung der Salivation, z.B. Rad.pyrethri,

...gerung der körpereigenen Abwehrkraft, z.B. Echinacea, und

- zur Verbesserung von Bekömmlichkeit, Stoffwechselleistung und Geschmack, z.B. Zingiber, Calamus, Curcuma und Angustura,

c) ätherische Oele und Resinoide

- mit durchblutungsfördernder Wirkung auf Zahnfleisch und Mundhöhle, z.B. Salbeiöl, Nelkenöl,

- zur Anregung der Speichelsekretion, z.B. Essence de piment, Nelkenöl,

- gegen Parodontose, z.B. Salbeiöl, Nelkenöl,

- zur Verbesserung der Bekömmlichkeit, der Stoffwechselleistung und des Geschmacks, z.B. Anisöl, Fenchelöl, Cassisöl,

- zur Aromatisierung, Erfrischung und Desodorierung, z.B. Pfefferminzöl, Krauseminzöl, Menthol, Eucalyptusöl und Citronenöl,

- zur Kompensierung der Nikotinwirkung nach dem Rauchen in geschmacklicher und gesundheitlicher Hinsicht, z.B. Pfefferminzöl, Menthol und Eucalyptusöl,

d) Vitamin in Form von Calcium-D-Pantothenat, sowie

e) Weichmacher und Binder in Form von nicht gärfähigen und nicht kariogenen zuckerähnlichen Verbindungen, zur Geschmacksverbesserung und Kariesverhütung, z.B. Sorbit und Glycerin.

6. Kaumasse nach einem der Ansprüche 1 bis 4, dadurch gekennzeichnet, dass sie folgende Bestandteile enthält:

| | | |
|---|---|---|
| a) | Kaubare Naturharze als Kaugummigrundmasse | 25,0 Gew.-% |
| b) | Knochenmehlverreibung | 5,0 Gew.-% |
| c) | Meersalz | 1,5 Gew.-% |
| d) | Calciumcarbonat | 5,0 Gew.-% |
| e) | Natriumbicarbonat | 0,2 Gew.-% |
| f) | Kräutertinktur | 7,0 Gew.-% |

0205133

|  | | |
|---|---|---|
| aus Tinct. ratanhiae | 55,0 | g |
| aus Tinct. echinacea | 8,8 | g |
| aus Tinct. benzoe | 8,8 | g |
| aus Tinct. myrrhae | 8,8 | g |
| aus Tinct. iridis | 8,9 | g |
| aus Tinct. symphyti | 12,0 | g |
| aus Tinct. nasturtii | 8,8 | g |
| aus Tinct. cochleariae | 8,8 | g |
| aus Tinct. salviae | 8,8 | g |
| aus Tinct. chamomillae | 8,8 | g |
| aus Tinct. zingiberis | 11,0 | g |
| aus Tinct. calami | 11,0 | g |
| aus Tinct. curcumae | 11,0 | g |
| aus Tinct. angusturae | 8,8 | g |
| aus Tinct. pyrethri comm. | 8,8 | g |
| aus Tinct. caryophylli | 11,0 | g |
| | 200,0 | g |

g) Calcium-D-Pantothenat          0,1 Gew.-%

h) ätherische Oele,          1,5 Gew.-%

|  | | |
|---|---|---|
| nämlich Pfefferminz-<br>öl Ia | 600 | g |
| Krauseminzöl | 25 | g |
| Menthol recrist DAB<br>42/44° | 120 | g |
| Eucalyptusöl span.<br>glob.rekt. 70/75 % | 50 | g |
| Anisöl amerik. | 50 | g |
| Fenchelöl span.süss<br>DAB | 50 | g |
| Salbeiöl span. EB | 20 | g |
| Nelkenöl gereinigt<br>80/85 % | 20 | g |
| Cassisöl rekt. 80/85% | 50 | g |
| Citronenöl ital. DAB | 5 | g |
| Essence de piment | 10 | g |
| | 1000 | g |

- 4 -

kariogener Zuckersatz,

| | |
|---|---|
| wie z.B. Sorbit, Mannit | 53,9 Gew.-% |
| k) Glycerin | 0,5 Gew.-% |
| 1) natürliche Farbstoffe | 0,3 Gew.-%. |

7. Kaumasse nach einem der Ansprüche 1 bis 4, dadurch gekennzeichnet, dass sie folgende Bestandteile enthält:

a) ein Konzentrat aus einer Knochenmehlverreibung, Meersalz, Calciumcarbonat und Natriumbicarbonat,

b) pflanzliche Wirkstoffe in homöopathischen Potenzen:

- gegen Krämpfe, bei Schwäche und Müdigkeit, z.B. Lobelia inflata = indischer Tabak,

- bei Erregungszuständen, Unruhe, Nervosität, z.B. Stramonium,

- bei Stress, gegen Magenkrämpfe, gegen Schmerzen, Melancholie und Angst, z.B. Belladonna,

- bei Uebererregbarkeit und Kopfdruck, z.B. Anhalonium-Lewinii,

c) ätherische Oele und Resinoide

- mit durchblutungsfördernder Wirkung, z.B. Salbeiöl, Nelkenöl,

- zur Anregung der Speichelsekretion und des Stoffwechsels, z.B. Essence de piment, Nelkenöl,

- zur Verbesserung der Bekömmlichkeit, der Stoffwechselleistung und des Geschmacks, z.B. Anisöl, Fenchelöl, Cassisöl,

- zur Aromatisierung, Erfrischung und Desodorierung, z.B. Pfefferminzöl, Krauseminzöl, Menthol, Eucalyptusöl und Citronenöl,

d) Vitamin in Form von Calcium-D-Pantothenat,

e) Weichmacher und Binder in Form von nicht gärfähigen zuckerähnlichen Verbindungen, zur Geschmacksverbesserung, z.B. Sorbit und Glycerin.

8. Kaumasse nach einem der Ansprüche 1 bis 4, dadurch gekennzeichnet, dass sie folgende Bestandteile
enthält:

| | | |
|---|---|---|
| a) | Kaubare Naturharze als Kaugummi-grundmasse | 25,0 Gew.-% |
| b) | Knochenmehlverreibung | 5,0 Gew.-% |
| c) | Meersalz | 1,5 Gew.-% |
| d) | Calciumcarbonat | 5,0 Gew.-% |
| e) | Natriumbicarbonat | 0,2 Gew.-% |
| f) | Pflanzliche Wirkstoffe: | 7,0 Gew.-% |

| | |
|---|---|
| Lobelia inflata | $D_{12}$ |
| Stramonium | $D_{12}$ |
| Belladonna | $D_{12}$ |
| Anhalonium | $D_{12}$ |
| aa | 75,0 g |

| | | |
|---|---|---|
| g) | Calcium-D-Pantothenat | 0,1 Gew.-% |
| h) | ätherische Oele, | 1,5 Gew.-% |

| | | |
|---|---|---|
| nämlich Pfefferminz-öl Ia | 600 | g |
| Krauseminzöl | 25 | g |
| Menthol recrist DAB 42/44° | 120 | g |
| Eucalyptusöl span. glob.rekt. 70/75 % | 50 | g |
| Anisöl amerik. | 50 | g |
| Fenchelöl span.süss DAB | 50 | g |
| Salbeiöl span. EB | 20 | g |
| Nelkenöl gereinigt 80/85 % | 20 | g |
| Cassisöl rekt. 80/85% | 50 | g |
| Citronenöl ital. DAB | 5 | g |
| Essence de piment | 10 | g |
| | 1000 | g |

| | | |
|---|---|---|
| i) | nicht gärfähiger Zuckerersatz, wie z.B. Sorbit, Mannit | 53,9 Gew.-% |
| k) | Glycerin | 0,5 Gew.-% |
| l) | natürliche Farbstoffe | 0,3 Gew.-%. |

aumasse nach einem der Ansprüche 1 bis 4, dadurch gekennzeichnet, dass sie folgende Bestandteile enthält:

a) ein Konzentrat aus einer Knochenmehlverreibung, Meersalz, Calciumcarbonat und Natriumbicarbonat;

b) pflanzliche und biologische Wirkstoffe in homöopathischen Potenzen:

- gegen Schwäche und zur Beruhigung, z.B. Ferrum phosphoricum,

- bei Depressionen und Müdigkeit, z.B. Kalium phosphoricum,

- bei zu schneller Ermüdung und Abgespanntheit, z.B. Semen colae,

- als Tonikum bei Schwächegefühl, z.B. Muira puama,

c) ätherische Oele und Resinoide

mit durchblutungsfördernder Wirkung, z.B. Salbeiöl, Nelkenöl,

zur Anregung der Speichelsekretion und des Stoffwechsels, z.B. Essence de piment, Nelkenöl,

zur Verbesserung der Bekömmlichkeit, der Stoffwechselleistung und des Geschmackes, z.B. Anisöl, Fenchelöl, Cassisöl,

zur Aromatisierung, Erfrischung und Desodorierung, z.B. Pfefferminzöl, Krauseminzöl, Menthol, Eucalyptusöl und Citronenöl,

d) Vitamin in Form von Calcium-D-Pantothenat, sowie e) Weichmacher und Binder in Form von nicht gärfähigen zuckerähnlichen Verbindungen zur Geschmacksverbesserung, z.B. Sorbit und Glycerin.

10. Kaumasse nach einem der Ansprüche 1 bis 4, dadurch gekennzeichnet, dass sie folgende Bestandteile enthält:

| | |
|---|---|
| a) Kaubare Naturharze als Kaugummigrundmasse | 25,0 Gew.-% |
| b) Knochenmehlverreibung | 5,0 Gew.-% |
| c) Meersalz | 1,5 Gew.-% |

| | |
|---|---|
| d) Calciumcarbonat | 5,0 Gew.-% |
| e) Natriumbicarbonat | 0,2 Gew.-% |
| f) Pflanzliche und biologische Wirkstoffe: | 7,0 Gew.-% |

| | |
|---|---|
| Ferrum phos. | $D_1$ |
| Kalium phos. | $D_1$ |
| Semen colae | $D_{12}$ |
| Muira puama | $D_{12}$ |
| aa | 75,0 g |

| | |
|---|---|
| g) Calcium-D-Pantothenat | 0,1 Gew.-% |
| h) ätherische Oele, | 1,5 Gew.-% |

| | | |
|---|---|---|
| nämlich Pfefferminz-öl Ia | 600 | g |
| Krauseminzöl | 25 | g |
| Menthol recrist DAB 42/44° | 120 | g |
| Eucalyptusöl span. glob.rekt. 70/75 % | 50 | g |
| Anisöl amerik. | 50 | g |
| Fenchelöl span.süss DAB | 50 | g |
| Salbeiöl span. EB | 20 | g |
| Nelkenöl gereinigt 80/85 % | 20 | g |
| Cassisöl rekt. 80/85% | 50 | g |
| Citronenöl ital. DAB | 5 | g |
| Essence de piment | 10 | g |
| | 1000 | g |

| | |
|---|---|
| i) nicht gärfähiger Zuckerersatz, wie z.B. Sorbit, Mannit | 53,9 Gew.-% |
| k) Glycerin | 0,5 Gew.-% |
| l) natürliche Farbstoffe | 0,3 Gew.-%. |

11. Verwendung der Kaumasse nach einem der Ansprüche 1 bis 10

a) als reinigendes Mund- und Zahnpflegemittel zum Schutz der Zähne, des Parodontiums und der Mundschleimhaut, und/oder

b) als biologisches und völlig unschädliches

- 8 -

Raucherentwöhnungsmittel, und/oder

c) als biologisches und völlig unschädliches Mittel zur Steigerung der Vitalität und der Leistungsfähigkeit.

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | DE-A-2 043 279 (K.-O. HEEDE) <br> * Seiten 1-11 * <br><br> --- <br><br> | 1-11 | A 61 K 7/16 <br> A 23 G 3/30 <br> A 61 K 7/26 <br> A 61 K 9/68 |
| A | DE-A-2 263 106 (DENTASAN et al.) <br><br> ----- | | |

| | RECHERCHIERTE SACHGEBIETE (Int. Cl 4) |
|---|---|
| | A 23 G |

Der vorliegende Recherchenbericht wurde für alle Patentansprüche erstellt.

| Recherchenort <br> DEN HAAG | Abschlußdatum der Recherche <br> 12-08-1986 | Prüfer <br> GUYON R.H. |
|---|---|---|

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82